(19) Europäisches Patentamt
European Patent Office
Office européen des brevets

(11) Publication number: 0 115 965 B1

(12) **EUROPEAN PATENT SPECIFICATION**

(45) Date of publication of patent specification: **10.05.89**

(51) Int. Cl.$^4$: **C 07 J 1/00,** C 07 J 17/00, C 07 J 51/00

(21) Application number: **84300734.5**

(22) Date of filing: **06.02.84**

(54) **Process for converting 17-keto-steroids to 16-beta-methyl-17-keto-steroids.**

(30) Priority: **09.02.83 US 465141**

(43) Date of publication of application: **15.08.84 Bulletin 84/33**

(45) Publication of the grant of the patent: **10.05.89 Bulletin 89/19**

(84) Designated Contracting States: **BE CH DE FR GB IT LI NL SE**

(56) References cited:
**US-A-2 883 401**

**GAZZETTA CHIMICA ITALIANA, vol.91, 1961, pages 672-685, Rome, IT; P. de RUGGIERI et al.: "Steroidi. - Nota IX. 16-metil-androstani. 16alpha- e 16bêta-metil-deidroepi-androsterone"**

**J. FRIED, J.A. EDWARDS: Org. Reactions in Steroid Chemistry, Van Nostrand Reinhold Comp. 1972, pages 88-91**

**The file contains technical information submitted after the application was filed and not included in this specification**

(73) Proprietor: **THE UPJOHN COMPANY
301 Henrietta Street
Kalamazoo, Michigan 49001 (US)**

(72) Inventor: **VanRheenen, Verlan Henry
c/o The Upjohn Company 301 Henrietta Street
Kalamazoo Michigan 49001 (US)**
Inventor: **Timko, Joseph Michael
c/o The Upjohn Company 301 Henrietta Street
Kalamazoo Michigan 49001 (US)**

(74) Representative: **Perry, Robert Edward et al
GILL JENNINGS & EVERY 53-64 Chancery Lane
London WC2A 1HN (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 115 965 B1

## Description

The invention relates to a process for converting 17-keto-steroids to the corresponding 16β-methyl-17-keto-steroids. The starting materials and products are respectively illustrated by the partial formulae I and IV in Chart A.

16β-methyl-17-keto-steroids are important starting materials in the production of 16β-methyl-corticoids. It is therefore desirable that the conversion process to which this invention relates should be conducted stereo-selectively, as far as possible, to give high yields.

Known processes for the conversion include those described in US—A—3039528 and US—A—3704253. Most of the processes produce some 16α-methyl isomer. Neef, J. Org. Chem. *43* (1978) 4679, determined that the thermodynamic equilibrium mixture of 16β-methyl/16α-methyl was 78.5/21.5 for 3β-acetoxy-16-methylandrost-5-en-17-one.

In particular, deRuggiere *et al.*, Gazz. Chim. Ital. *91* (1961) 672 (see particularly p.678) disclose a three-step process for the conversion of a 17-keto-steroid (I) to the corresponding 16β-methyl-117-keto-steroid (IV). The process involves glyoxalation, methylation and deglyoxation. While deRuggieri *et al.* do not disclose the 16β-methyl/16α-methyl product ratio, a low yield (45.8%) was obtained.

According to the present invention, a 16β-methyl-17-keto-steroid of partial formula IV is prepared by the three steps illustrated in Chart A, the first step involving reaction with a $C_{16}$ activating agent in the presence of an alkali metal alkoxide, thereby producing an anion of a 16-substituted steroid of partial formula II, the second step comprising reaction of the anion with a methylating agent, and the third step comprising reaction of the resultant 16-methyl-16-substituted steroid of partial formula III with a strong base in a solvent containing an alcohol.

The process of the present invention can provide improved yield of the 16β-methyl product, if necessary by protecting the $C_3$ position. A 16β-methyl/16α-methyl ratio of greater than 90/10, and up to 95/5, can be achieved.

The invention can be conducted for conversion of steroids having the partial formulae A and B. Where necessary, a desirable $C_3$ functionality is protected. With respect formula A, the 3-keto group is protected as the enol ether of partial formula Aa or ketal of partial formula Ab. No protection is necessary in the case of the 1,4-dien-3-ones of formula B.

The protecting group $R_3$ is $C_{1-5}$ alkyl, 2-tetra hydropyranyl or 2-tetrahydrofuranyl, and usually $C_{1-5}$ alkyl (including isomeric forms). In formula Aa, $R_3$ is preferably methyl or ethyl. In formula Ab, the two groups $R_3$ may together form a $C_{1-5}$ alkylene, preferably ethylene, radical.

Enol ethers and ketals may be prepared by well known methods. The preparation of ketals is described in "Steroid Reactions", ed. Carl Djerassi, Holden-Day, San Francisco (1963) 11-14. Enol ether preparation is described in "Steroid Reactions", supra, 42—45; J. Org. Chem. *26* (1961) 3925 and US—A—3516991 (Preparation 1).

In the first step of the process of the present invention, the 17-keto-steroid (I) is reacted with a $C_{16}$ activating agent of the formula R'O—CO—R, in which R is H, OR' or COOR' and R' is $C_{1-5}$ alkyl, with an alkali metal alkoxide in an inert organic solvent at a temperature from about −20° to the reflux temperature of the inert organic solvent. It is prefered that the $C_{16}$ activating agent be di($C_{1-3}$)alkyloxalate, more preferably dimethyl or diethyl oxalate. The alkali metal alkoxides include lithium, sodium and potassium with sodium being preferred. The alkyl portion of the alkoxide includes alkyl of preferably 1 through 4 carbon atoms. The preferred alkali metal alkoxides are sodium methoxide and sodium ethoxide. Inert organic solvents include methylene chloride, THF, diethyl ether and dimethoxyethane. The preferred temperature range is from about 0° to about 25°. The 16-substituted steroid (II) can be isolated and purified. In performing the next step, methylation, it is not necessary to purify the 16-substituted steroid (II) and it is preferable to perform the next step without isolation and purfication of its 16-substituted steroid (II). The 16-substituted steroid (II) is actually present as the alkali metal salt of an anion of the $C_3$ protected form (II). If it is desired to isolate the 16-substituted steroid (II), one would acidify so as to transform the anion to the non-charged 16-substituted steroid (II). Before performing the next step, methylation, the 16-substituted steroid (II) would again have to be subjected to alkaline conditions.

The 16-substituted steroid is then methylated to produce a 16-methyl-16-substituted steroid (III). Methylation is performed by reacting the alkali metal salt of its $C_3$ protected steroid (II) with a methylating agent, preferably a methyl halide in an inert organic solvent from about −20° to the reflux temperature of its inert organic solvent or the reaction vessel may be sealed and run at temperatures up to about 150°C. Methyl halides include methyl iodide and methyl bromide with methyl iodide preferred. Inert organic solvents include acetone, THF, diethyl ether, methylethylketone and dimethoxyethane; acetone is the preferred organic solvent. Methylene chloride retards the methylation reaction. Neutralization of the excess sodium methylate with an acid such as acetic acid and addition of potassium carbonate reduces by-product formation during methylation. It is preferred to use an excess of methylating agent. With methyl iodide preferred reaction conditions are 1.1—5.0 equivalents at 50—75° in acetone in a sealed reaction vessel. A full 5.0 equivalents of methyl iodide are used to promote methylation at 55° in 20—24 hours. If reaction conditions are chosen such that methylation takes 48 hours, the reaction shows considerable hydrolysis of the 3-enol ester of the $\Delta^4$-3-keto (A) steroids. When methyl iodide is used as the methylating agent, methyl bromide can be added to the reaction mixture when the reaction is complete. The excess methyl iodide and

organic solvent are removed under reduced pressure to give the 16-methyl-16-substituted steroid (III) which can be purified if desired. However, it is not necessary or even desirable to purify its 16-methyl-16-substituted steroid (III) but rather it is preferable to perform the third and last step directly on the impure 16-methyl-16-substituted steroid (III).

The 16-methyl-16-substituted (III) is transformed to the desired 16β-methyl-17-keto steroid (IV) by reaction with a stong base in an alcoholic solvent (ROH where R is alkyl of 1 through 5 carbon atoms). Strong bases include, for example, alkali metal (lithium, sodium, potassium) alkoxides (where the alkyl portion of the alkoxide includes alkyl of 1 through 4 carbon atoms), alkali metal (lithium, sodium, potassium) hydroxides and alkali metal (lithium, sodium, potassium) carboantes. It is preferable to use no more than 1 equivalent of base, with 1.0 equivalents being preferred. The preferred system is sodium methoxide in methanol. Water can be present in the system. Alternatively, an inert solvent such as THF, diethyl ether, methylene chloride or dimethoxyethane, or mixtures thereof containing one of the above-mentioned bases and an alcohol co-solvent may be used.

It is realized that in this third step, the deglyoxalation reaction, both 16β-methyl and 16α-methyl isomers are produced. The reaction temperature is critical to obtaining an isomer ratio of greater than or equal to 90% of the 16β-methyl (desired) to the 16α-methyl (undesired) ethylated -17-keto steroid (IV). At very low temperatures unwanted side reactions can occur and at high temperatures epimerization of the 16β-methyl group occurs. Temperatures between about −20° and about 25° are preferable, with about −5° to about 10° being more preferred. The optimum temperature for each reaction will depend on the particular 16-methyl-16-substituted steroid (III), the particular strong base and the particular solvent as is well known to those skilled in the art and therefore may vary slightly from the −5° to 10° generally preferred range.

With the $\Delta^4$-3-keto steroid (A) its 16α-methyl-17-keto product is in the $C_3$ protected form. These $C_3$ protected steroids can either be used in their $C_3$ protected forms as discussed below or can be readily transformed to the free or unprotected form by means well known to those skilled in the art.

The 16β-methyl-17-keto steroids (IVA—B) are useful intermediates in the production of commercially valuable 16β-methyl anti-inflammatory corticoids such as betamethasone, meprednisone, beclomethasone and diflorasone diacetate; see US—A—4041055, US—A—4216159, US—A—4284827 and US—A—4342702. A $\Delta^4$-3-keto-16β-methyl-17-keto steroid (IVA) can be dehydrogenated by means well known to those skilled in the art to the corresponding $\Delta^{4-4}$-3-keto-16β-methyl- 17-keto steroid (IVB).

DEFINITIONS

The definitions and explanations below are for the terms as used throughout the specification.

All temperatures are in degrees Centigrade.

TLC refers to thin-layer chromatography.

THF refers to tetrahydrofuran.

NMR refers to nuclear (proton) magnetic resonance spectroscopy; chemical shifts are reported in ppm (δ) downfield from tetramethylsilane.

$R_6$ is H or F.

$R_{11}$ is a hydrogen atom, α-OH or β-OH (but is H when the 9,11-bond is a double bond).

---- is a single or double bond.

The following Examples 1 to 3 and 4 to 6 respectively illustrate the three process steps of the present invention.

### Example 1

3-Methoxy-16-(methyloxalyl)androsta-3,5,9(11)-trien-17-one sodium salt (IIAa)

3-Methoxyandrosta-3,5,9(11)-trien-17-one (IAa) US—A—3516991, (29.84 g), methylene chloride (200 ml) and diethyl oxalate (16.1 g) are cooled to 0°. Solid sodium methoxide (6.5 g) is added. The cooling bath is removed and the mixture stirred at 20—25°. After one hour, TLC indicates some starting material remains. Additional sodium methoxide (1.08 g) is added. After an additional hour, TLC shows no starting material remained. Sodium bicarbonate (3.6 g) is added, the solution stirred for 10 minutes and the solvent removed at a reduced pressure to give the title compound.

### Example 2

3-Methoxy-16-methyl-16-(methyloxalyl)androsta-3,5,9(11)-trien-17-one (IIIAa)

Acetone (200 ml) is added to 3-methoxy-16-(methyloxalyl)androsta-3,5,9(11)-trien-17-one sodium salt (IIAa, Example 1) to produce a thin slurry. Methyl iodide (70.95 g) is added and the flask stoppered with a rubber septum sealed with parafilm and heated to 65°. After 18 hours, the mixture is cooled to 20—25°C. TLC shows no remaining glyoxalate starting material. In order to regenerated the methyl iodide, methyl bromide in acetone (1 equivalent, 1.0 M, 100 ml) is added and mixture stirred for 15 minutes. The organic solvent and methyl iodide are removed under reduced pressure to give the title compound as a solid.

### Example 3

3-Methoxy-16β-methylandrosta-3,5,9(11)-trien-17-one (IVAa)

To the crude methylated glyoxalate (IIIAa, Example 2) as a solid is added methanol (300 ml), and the resulting slurry is cooled to −10° utilizing overhead stirring. A methanolic solution of sodium methoxide is

then added (25%, 23 ml). After 15 minutes at −10°, reaction is complete as measured by TLC. The slurry is poured into water (300 ml) at 5° containing acetic acid (6.9 ml); the mixture is filtered and the solid washed with water (100 ml) and dried under reduced pressure at 50° to give the title compound, m.p. 124—129°; NMR ($CDCl_3$) 0.83, 1.13, 1.18, 3.53, 5.13, 5.27 and 5.48 δ.

Example 4

3-Methoxy-16-(methyloxalyl)androsta-3,5-dien-17-one sodium salt (IIIAa)

Methylene chloride (133 ml), diethyl oxalate (10.7 g) and 3-methoxyandrosta-3,5-dien-17-one (IAa, 20 g) are mixed and cooled to 0°. Solid sodium methoxide (4.3 g) is added, the cooling bath removed and the reaction stirred at 20—25° overnight. Sodium methoxide (0.71 g) is added and the mixture is stirred for 2.75 hours. Additional sodium methoxide (0.71 g) is added and the mixture stirred for an additional 2 hours, whereupon sodium methoxide (0.35 g) is added followed by diethyl oxalate (1.076 g). The mixture is stirred for an additional 30 minutes, following which sodium carbonate (1.7 g) is added; the mixture is stirred for an additional 30 minutes after which the methylene chloride is removed under reduced pressure to give the title compound.

Example 5

3-Methoxy-16-methyl-16-(methyloxalyl)androsta-3,5-dien-17-one (IIIAa)

Acetone (133 ml) is added to 3-methoxy-16-(methyloxalyl)androsta-3,5-dien-17-one sodium salt (II, Example 4), followed by methyl iodide (20.5 ml). The flask is stoppered by rubber septum and sealed with parafilm and heated to 55° for 17 hours. The reaction is complete as measured by TLC. The mixture is cooled to 20—25° and methyl bromide in acetone (1.0 equivalent) is added. The reaction mixture is stirred for 10 minutes and then concentrated under reduced pressure to give the title compound.

Example 6

3-Methoxy-16β-methylandrosta-3,5-dien-17-one (IVAa)

Methanol (133 ml) is added to 3-methoxy-16-methyl-16-(methyloxalyl)androsta-3,5-dien-17-one (III, Example 5). The slurry is cooled to 0° with an ice-bath and one equivalent of methanolic sodium methoxide (25%, sodium methoxide/methanol, 15.1 ml) is added. After one hour, the reaction is complete as measured by TLC. The mixture is poured into ice-water (135 ml) containing acetic acid (4.9 ml). The solid material is obtained by filtration and dried under reduced pressure at 50° overnight to give the title compound. m.p. 149—167°; NMR ($CDCl_3$), 0.87, 1.00, 1.22, 3.56, 5.12 and 5.23 δ.

CHART A

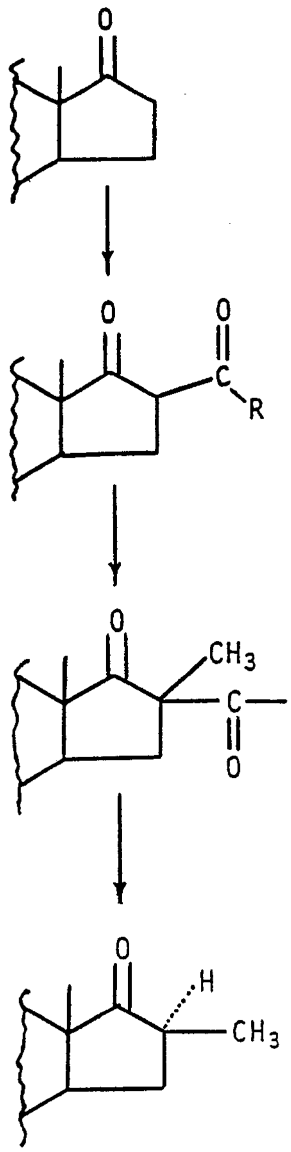

(I)

(II)

(III)

(IV)

(A)

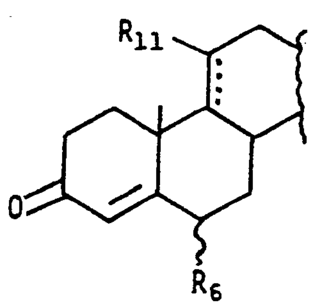

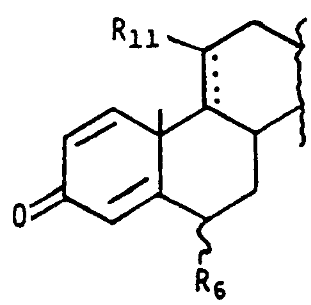

(B)

(Aa)

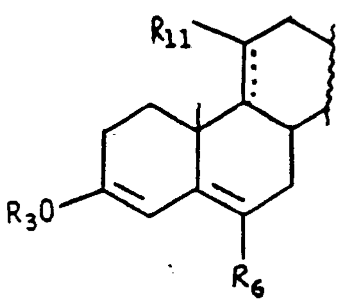

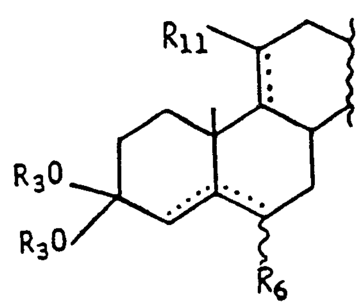

(Ab)

**Claims**

1. A process for preparing a 16β-methyl-17-keto-steroid of the formula

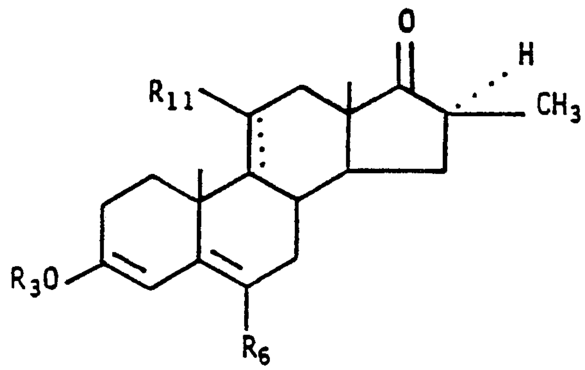

(IVAa)

which comprises (1) contacting a 17-keto-steroid of the formula

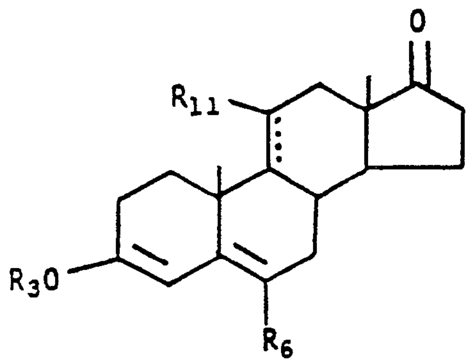

(IAa)

with a $C_{16}$ activating agent of the formula R'O—CO—R, in the presence of an alkali metal alkoxide, to give an anion of a 16-substituted steroid of the formula

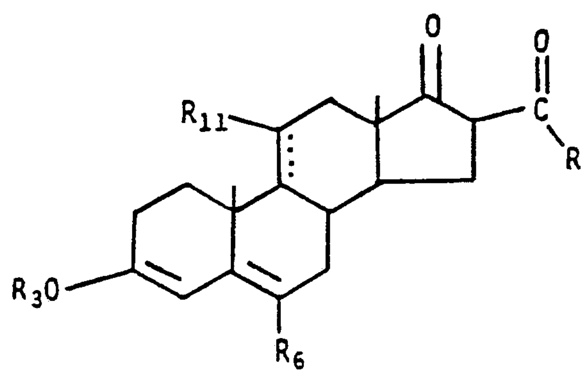

(IIAa)

(2) contacting the anion with a methylating agent, to give a 16-methyl-16-substituted-steriod of the formula

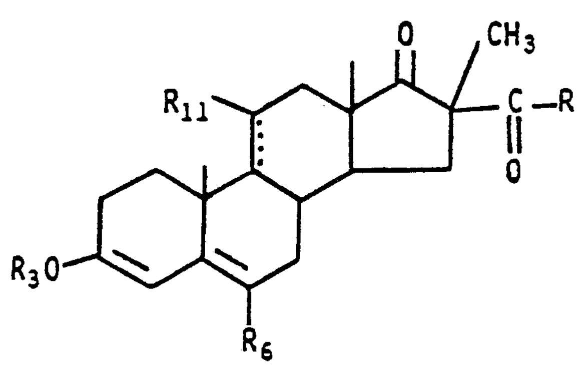

(IIIAa)

and (3) contacting the 16-methyl-16-substituted steroid with a strong base selected from $C_{1-4}$ alkali metal

alkoxides, alkali metal hydroxides and alkali metal carbonates, in a solvent containing an alcohol;
wherein R is H, OR' or COOR' and R' is $C_{1-5}$ alkyl;
$R_3$ is $C_{1-5}$ alkyl, 2-tetrahydrofuranyl or 2-tetrahydropyranyl;
$R_6$ is H or F.
$R_{11}$ is H, α-OH or β-OH (but is H when the 9,11-bond is a double bond); and
---- is a single or double bond.

2. A process for preparing a 16β-methyl-17-keto-steroid of the formula

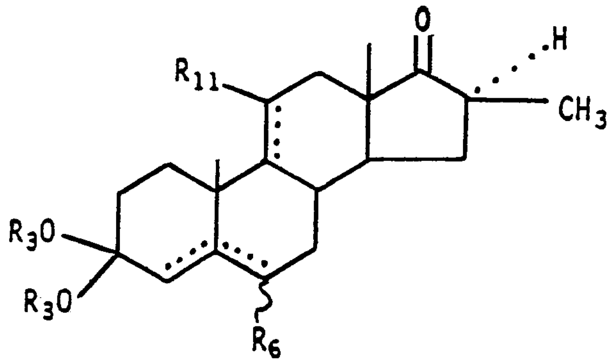

(IVAb)

which comprises (1) contacting a 17-keto-steroid of the formula

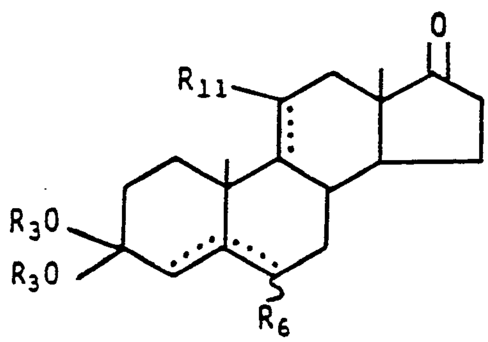

(IAb)

with a $C_{16}$ activating agent as defined in claim 1, in the presence of an alkali metal alkoxide, to give an anion of a 16-substituted steroid of the formula

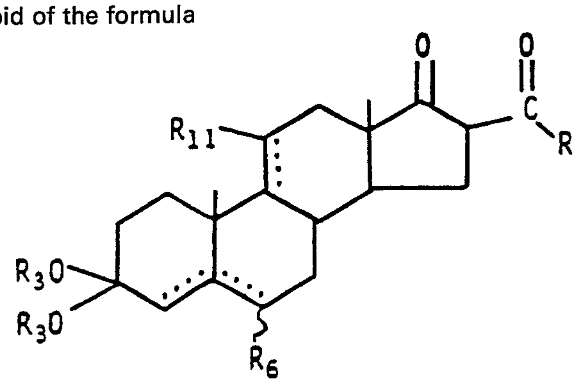

(IIAb)

(2) contacting the anion with a methylating agent, to give a 16-methyl-16-substituted-steroid of the formula

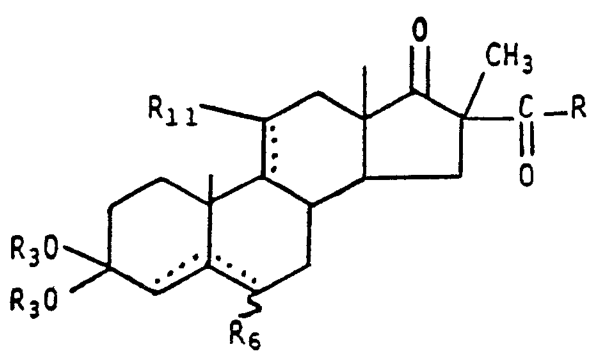

(IIIAb)

and (3) contacting the 16-methyl-16-substituted steroid with a strong base as defined in claim 1 in a solvent containing an alcohol; wherein R, $R_3$ (or two $R_3$'s together are alkylene), $R_6$ (~ indicates that $R_6$ may be α-F or β-F, when the 5,6-bond is a single bond), $R_{11}$ and .... (the 4,5- and 5,6-bonds may not both be double bonds) are as defined in claim 1.

3. A process for preparing a 16β-methyl-17-keto-steroid of the formula

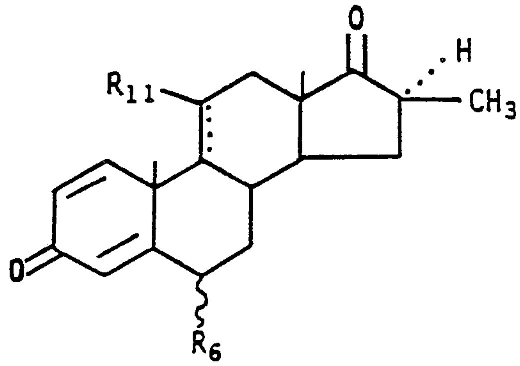

(IVB)

which comprises (1) contacting a 17-keto-steroid of the formula

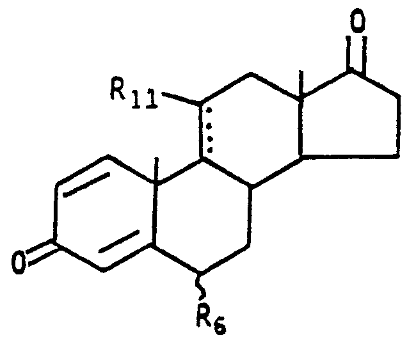

(IB)

with a $C_{16}$ activating agent as defined in claim 1, in the presence of an alkali metal alkoxide, to give an anion of a 16-substituted steroid of the formula

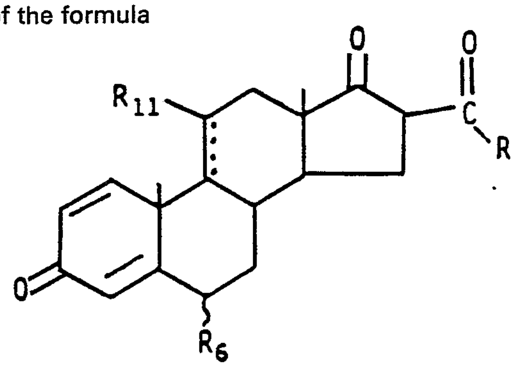

(IIB)

(2) contacting the anion with a methylating agent, to give a 16-methyl-16-substituted-steroid of the formula

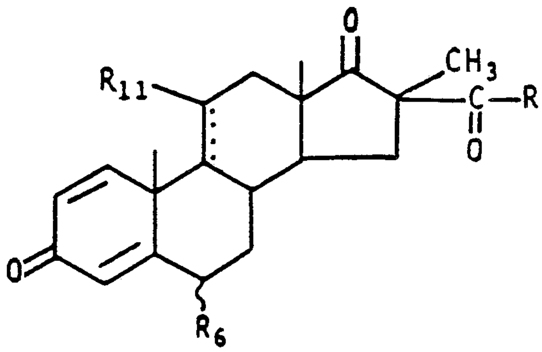

(IIIB)

and (3) contacting the 16-methyl-16-substituted steroid with a strong base as defined in claim 1 in a solvent

containing an alcohol; wherein R, $R_6$, $R_{12}$ and ....are as defined in claim 1, and ~ indicated α- or β configuration.

4. A process according to any preceding claim, wherein the alkali metal alkoxide is a lithium, sodium or potassium $C_{1-4}$ alkoxide.

5. A process according to any preceding claim, wherein the methylating agent is methyl iodide or methyl bromide.

6. A process according to any preceding claim, wherein the $C_{16}$ activating agent is a di($C_{1-3}$ alkyl) oxalate.

7. A process according to any preceding claim, wherein step (3) is conducted at −20 to 25°C.

8. 3-Methoxy-16-methyloxalyl-16-methylandrosta-3,5,9(11)-trien-17-one, or 3-methoxy-16-methyl-oxalyl-16-methylandrosta-3,5-dien-17-one.

**Patentansprüche**

1. Ein Verfahren zum Herstellen eines 16β-Methyl-17-Keto-Steroids der Formel

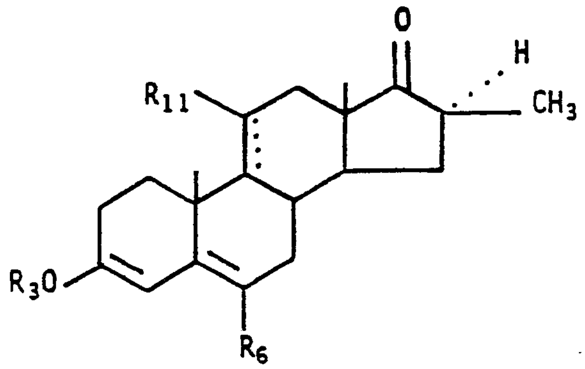

(IVAa)

umfassend (1) das Inkontaktbringen eines 17-Keto-Steroids der Formel

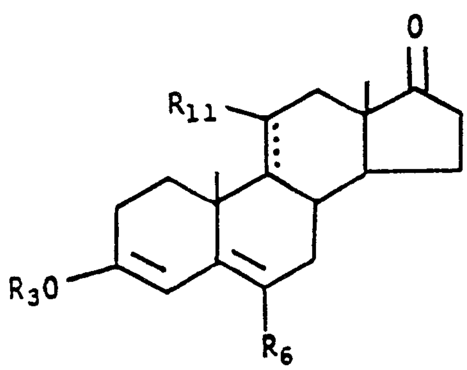

(IAa)

mit einem $C_{16}$ Aktiviermittel der Formel R'—O—CO—R, in Gegenwart eines Alkalimetallalkoxids, um ein Anion eines 16-substituierten Steroids der Formel

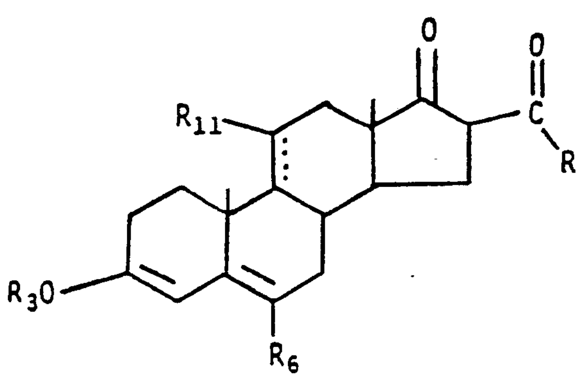

(IIAa)

zu erzielen, (2) dans Inkontaktbringen des Anions mit einem Methyliermittel, um ein 16-Methyl-16-substituiertes Steroid der Formel

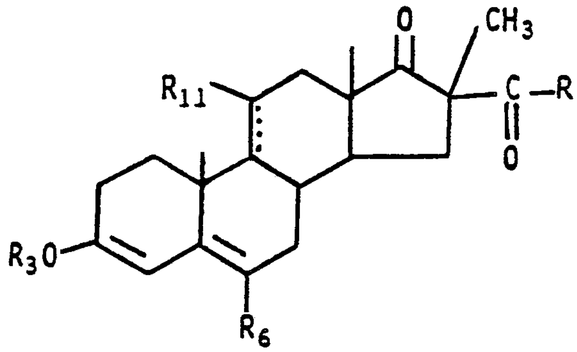

(IIIAa)

zu erzielen und (3) das Inkontaktbringen des 16-Methyl-16-substituierten Steroids mit einer starken aus der Gruppe $C_{1-4}$ Alkalimetallalkoxide, Alkalimetallhydroxide und Alkalimetallkarbonate ausgewählten Base in einem Alkohol enthaltenden Lösungsmittel;

worin R H, OR' oder COOR' und R' $C_{1-5}$ Alkyl ist;

$R_3$ $C_{1-5}$ Alkyl, 2-Tetrahydrofuranyl oder 2-Tetrahydropyranyl ist;

$R_6$ H oder F ist;

$R_{11}$ H, α-OH oder β-OH ist (aber H wenn die 9,11-Bindung eine Doppelbindung ist); und

.... eine einfache oder doppelte Bindung ist.

2. Ein Vefahren zum Herstellen eines 16β-Methyl-17-Ketosteroids der Formel

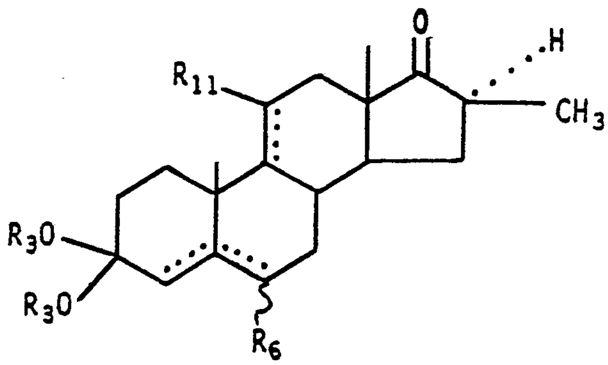

(IVAb)

umfassend (1) das Inkontaktbringen eines 17-Keto-Steroids der Formel

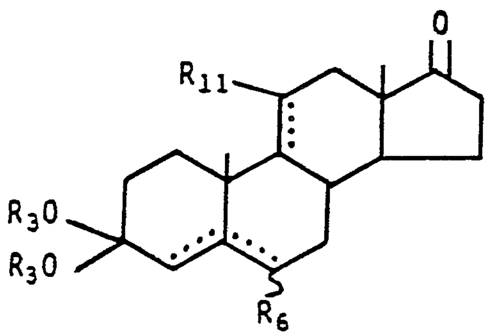

(IAb)

mit einem $C_{16}$ Aktiviermittel, wie in Anspruch 1 definiert, in Gegenwart eines Alkalimetallalkoxids, um ein Anion eines 16-substituierten Steroids der Formel

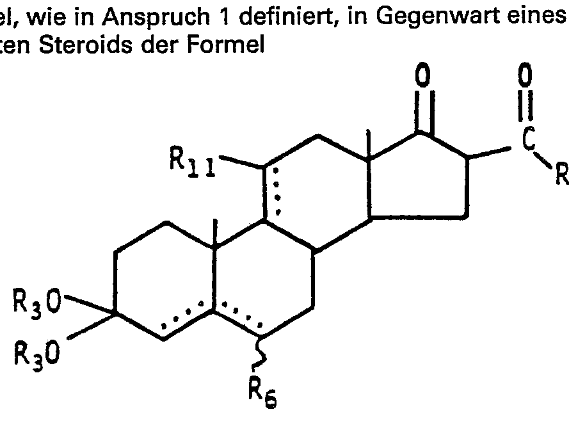

(IIAb)

zu erzielen, (2) das Inkontaktbringen des Anions mit einem Methyliermittel, um ein 16-Methyl-16-substituiertes Steroid der Formel

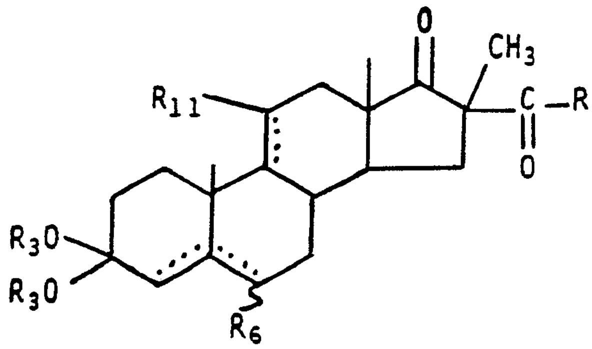

(IIIAb)

zu erzielen, und (3) das Inkontaktbringen des 16-Methyl-16-substituierten Steroids mit einer starken Base, wie in Anspruch 1 definiert, in einem einen Alkohol enthaltenden Lösungsmittel; worin R, $R_3$ (oder zwei $R_3$' gemeinsam Alkylen sind), $R_6$ (~ gibt an daß $R_6$ α-F oder β-F sein kann, wenn die 5,6-Bindung eine einfache Bindung ist), $R_{11}$ und .... (die 4,5- und 5,6-Bindungen können nicht beide Doppelbindungen sein) wie in Anspruch 1 definiert sind.

3. Ein Verfahren zum Herstellen eines 16β-Methyl-17-keto-Steroids der Formel

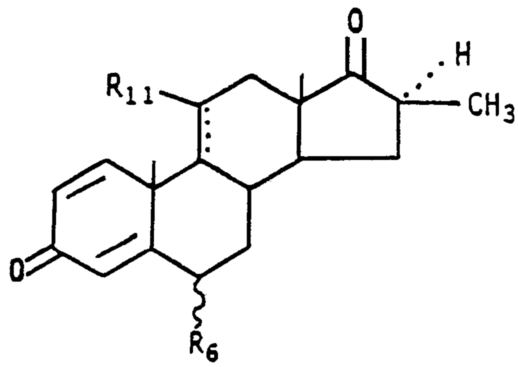

(IVB)

umfassend (1) das Inkontaktbringen eines 17-Keto-Steroids der Formel

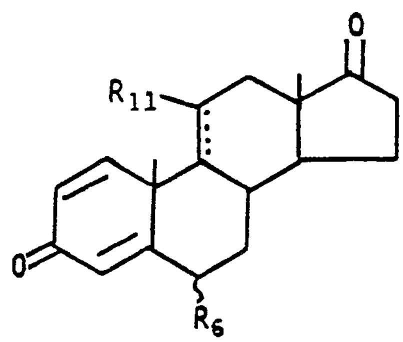

(IB)

mit einem $C_{16}$ Aktiviermittel, wie in Anspruch 1 definiert, in Gegenwart eines Alkalimetallalkoxids, um ein Anion eines 16-subsitituierten Steroids der Formel

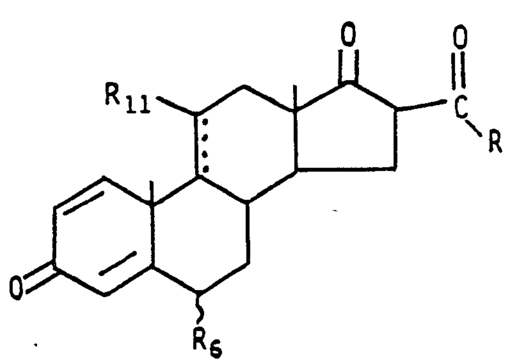

(IIB)

zu erzielen, (2) das Inkontaktbringen des Anions mit einem Methyliermittel, um ein 16-Methyl-16-substituiertes Steroid der Formel

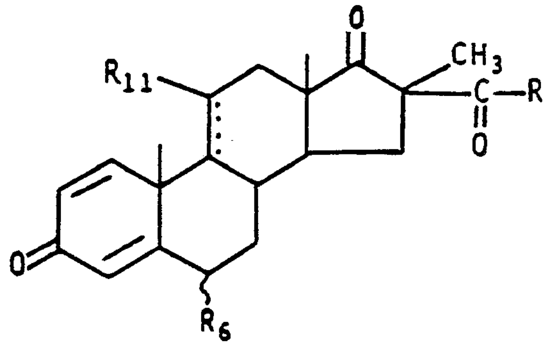

(IIIB)

zu erzielen, und (3) das Inkontaktbringen des 16-Methyl-16-substituierten Steroids mit einer starken Base, wie in Anspruch 1 definiert, in einem einen Alkohol enthaltenden Lösungsmittel; worin R, $R_6$, $R_{11}$ und wie in Anspruch 1 definiert sind und ~ eine α- oder β-Konfiguration bezeichnet.

4. Ein Verfahren nach einem der vorstehenden Ansprüche, bei dem das Alkalimetallalkoxid ein Lithium-, Natrium- oder Kalium $C_{1-4}$ Alkoxid ist.

5. Ein Verfahren nach einem der vorstehenden Ansprüche, bei dem das Methyliermittel Methyljodid oder Methylbromid ist.

6. Ein Verfahren nach einem der vorstehenden Ansprüche, worin das $C_{16}$ Aktiviermittel ein di($C_{1-3}$ Alkyl) Oxalat ist.

7. Ein Verfahren nach einem der vorstehenden Ansprüche, bei dem Schritt (3) bei −20 bis 25°C durchgeführt wird.

8. 3-Methoxy-16-Methyloxalyl-16-Methylandrosta-3,5,9(11)-Trien-17-On oder 3-Methoxy-16-Methyloxalyl-16-Methylandrosta-3,5-Dien-17-On.

**Revendications**

1. Procédé de préparation d'un 16β-méthyl-17-céto-stéroïde de formule

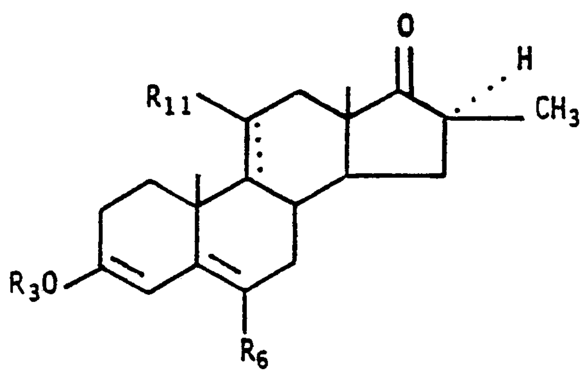

(IVAa)

qui consiste (1) à faire entrer un 17-céto-stéroïde de formule

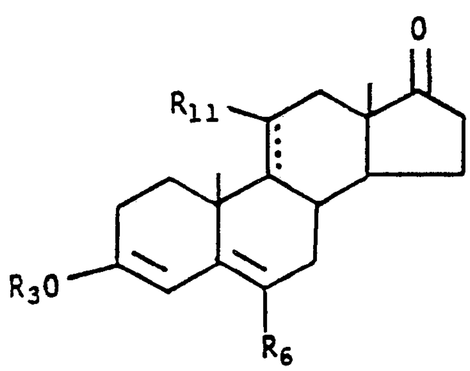

(IAa)

en contact avec un agent d'activation en position $C_{16}$ de formule R'O—CO—R en présence d'un alcoolate de métal alcalin pour former un anion d'un stéroïde 16-substitué de formule

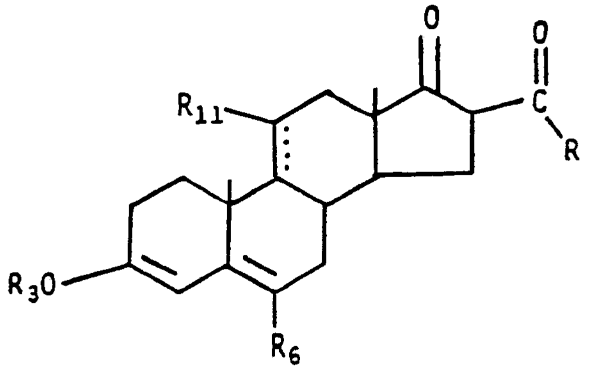

(IIAa)

(2) à faire entrer l'anion en contact avec un agent de méthylation pour obtenir un stéroïde 16-méthyl-16-substitué de formule

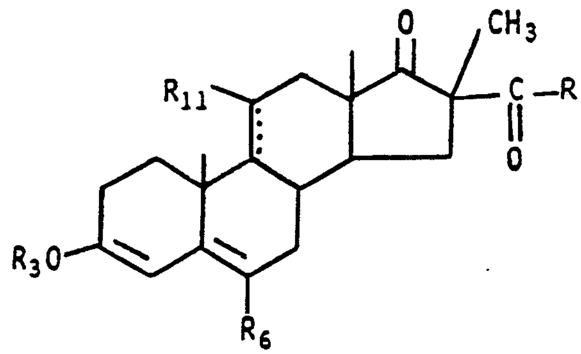

(IIIAa)

et (3) à faire entrer le stéroïde 16-méthyl-16-substitué en contact avec une base forte choisie entre des alcoolates de métaux alcalins en $C_1$ à $C_4$, des hydroxydes de métaux alcalins et des carbonates de métaux alcalins, dans un solvant contenant un alcool;

formules dans lesquelles R représente H, un groupe OR' ou COOR' et R' est un groupe alkyle en $C_1$ à $C_5$;

$R_3$ est un groupe alkyle en $C_1$ à $C_5$, 2-tétrahydrofurannyle ou 2-tétrahydropyrannyle;

$R_6$ représente H ou F;

$R_{11}$ représente H, un groupe α-OH ou β-OH (mais représente H lorsque la liaison 9,11 est une double liaison); et

.... est une liaison simple ou double.

2. Procédé de préparation d'un 16β-méthyl-17-céto-stéroïde de formule

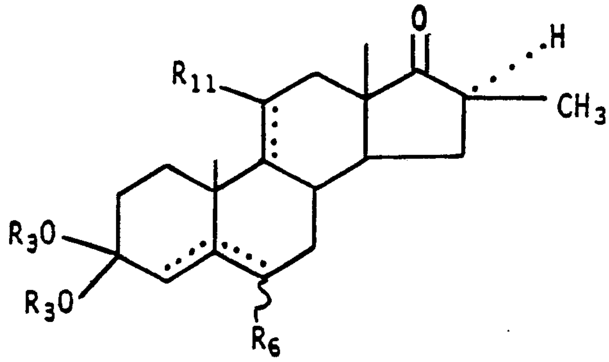

(IVAb)

qui consiste (1) à faire entrer un 17-céto-stéroïde de formule

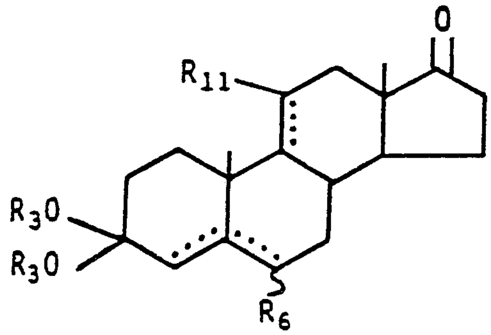

(IAb)

en contact avec un agent d'activation en position $C_{16}$ tel que défini dans la revendication 1, en présence d'un alcoolate de métal alcalin, pour former un anion d'un stéroïde 16-substitué de formule

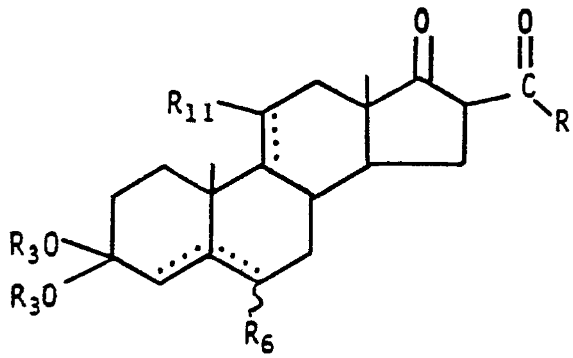

(IIAb)

(2) à faire entrer l'anion en contact avec un agent de méthylation pour obtenir un stéroïde 16-méthyl-16-substitué de formule

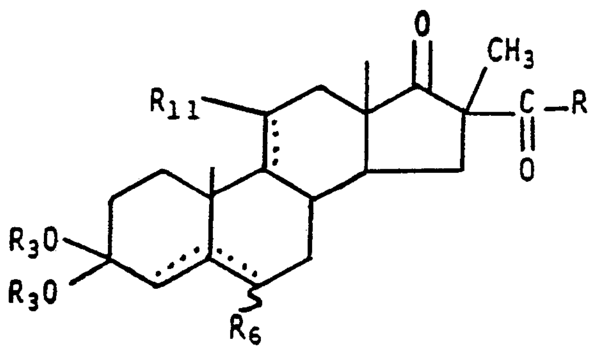

(IIIAb)

et (3) à faire entrer le stéroïde 16-méthyl-16-substitué en contact avec une base forte telle que définie dans la revendication 1 dans un solvant contenant un alcool; formules dans lesquelles R, $R_3$ (ou bien les deux groupes $R_3$ forment ensemble un groupe alkylène), $R_6$ (~ signifie que $R_6$ peut représenter α-F ou β-F lorsque la liaison en 5,6 est une liaison simple), $R_{11}$ et <u>....</u> (les liaisons en 4,5 et 5,6 ne peuvent pas toutes deux être des doubles liaisons) ayant les définitions données dans la revendication 1.

3. Procédé de préparation d'un 16β-méthyl-17-céto-stéroïde de formule

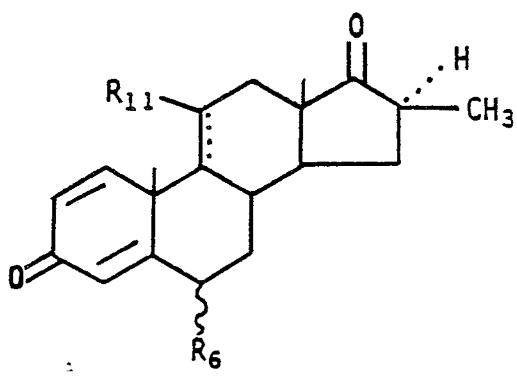

(IVB)

qui consiste (1) à faire entrer un 17-céto-stéroïde de formule

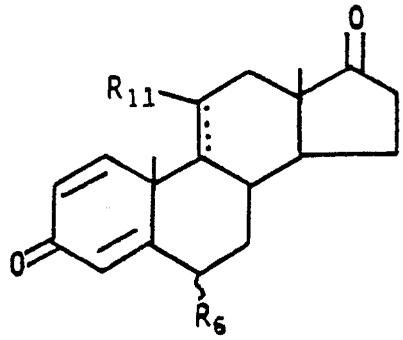

(IB)

en contact avec un agent d'activation en position $C_{16}$ tel que défini dans la revendication 1 en présence d'un alcoolate de métal alcalin pour former un anion d'un stéroïde 16-substitué de formule

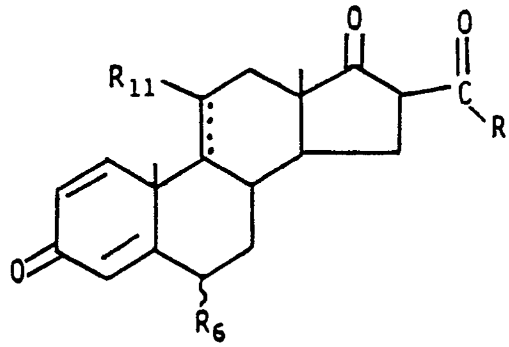

(IIB)

(2) à fiaire entrer l'anion en contact avec un agent de méthylation pour obtenir un stéroïde 16-méthyl-16-substitué de formule

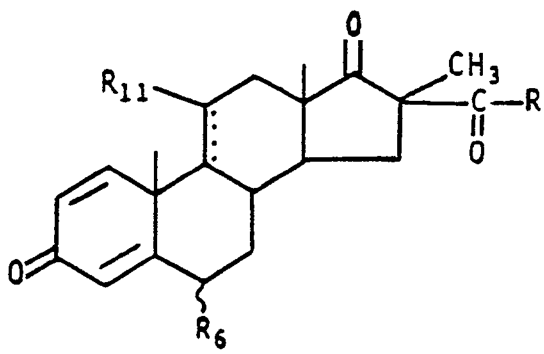

(IIIB)

et (3) à faire entrer le stéroïde 16-méthyl-16-substitué en contact avec une base forte telle que définie dans la revendication 1 dans un solvant contenant un alcool; formules dans lesquelles R, $R_6$, $R_{11}$ et .... sont tels que définis dans la revendication 1 et ~ indique la configuration α ou β.

4. Procédé suivant l'une quelconque des revendications précédentes, dans lequel l'alcoolate de métal alcalin est un alcoolate en $C_1$ à $C_4$ de lithium, sodium ou potassium.

5. Procédé suivant l'une quelconque des revendications précédentes, dans lequel l'agent de méthylation est l'iodure de méthyle ou le bromure de méthyle.

6. Procédé suivant l'une quelconque des revendications précédentes, dans lequel l'agent d'activation $C_{16}$ est un oxalate de di-(alkyle en $C_1$ à $C_3$).

7. Procédé suivant l'une quelconque des revendications précédentes, dans lequel l'étape (3) est conduite à une température de −20 à 25°C.

8. La 3-méthoxy-16-méthyloxalyl-16-méthylandrosta-3,5,9(11)-triène-17-one ou la 3-méthoxy-16-méthyloxalyl-16-méthylandrosta-3,5-diène-17-one.